(19) Europäisches Patentamt
European Patent Office
Office européen des brevets

(11) EP 1 077 064 A1

(12) **DEMANDE DE BREVET EUROPEEN**

(43) Date de publication:
**21.02.2001 Bulletin 2001/08**

(51) Int Cl.[7]: **A61K 7/48**, A61K 35/78

(21) Numéro de dépôt: **00402277.8**

(22) Date de dépôt: **11.08.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **16.08.1999 FR 9910511**

(71) Demandeur: **Desjonqueres, Stéphane**
**78600 Maisons-Laffitte (FR)**

(72) Inventeur: **Desjonqueres, Stéphane**
**78600 Maisons-Laffitte (FR)**

(74) Mandataire: **Giraud, Françoise et al**
**Cabinet Beau de Loménie**
**158, rue de l'Université**
**75340 Paris Cedex 07 (FR)**

# (54) Utilisation de lipides peroxydés comme agants destinés à former un film lipidique sur la peau

(57) L'invention concerne l'utilisation pour la fabrication d'une composition à usage topique destinée à former un film lipidique sur la peau, de lipides peroxydés en tant qu'agents filmogènes lipidiques.

Elle s'applique tout particulièrement dans tous les cas où l'on cherche à former un film destiné à protéger la surface de la peau, par exemple en vue d'améliorer la cicatrisation d'une plaie, de traiter des brûlures légères ou des érythèmes légers, en particulier dans le traitement de l'érythème solaire ainsi que dans le traitement de la dermite fessière du nourrisson.

L'invention permet également d'améliorer l'efficacité d'un massage par formation d'un film lipidique à la surface de la partie du corps à masser.



Printed by Jouve, 75001 PARIS (FR)

## Description

La présente invention concerne une nouvelle utilisation des lipides peroxydés comme agents destinés à former un film lipidique sur la peau.
A l'heure actuelle, il existe une recherche intense pour mettre au point des produits permettant d'améliorer une condition pathologique par la seule intervention d'une action mécanique, c'est-à-dire sans action pharmacologique.
A titre d'exemple de tels produits, on peut citer des produits formulés de façon à créer, à la manière d'un pansement, une barrière isolante à la surface d'une plaie de façon à permettre la cicatrisation naturelle de cette plaie en l'isolant du milieu extérieur.
Un autre exemple de domaines où l'on recherche des produits agissant sur une condition douloureuse uniquement par un effet mécanique est celui du massage thérapeutique. En effet, il est bien connu qu'un certain nombre de conditions douloureuses ou pathologiques répondent favorablement à l'action d'un massage. Toutefois, le massage est d'autant plus efficace qu'il s'effectue dans un environnement mécanique favorable, favorisant en particulier la glisse des mains du masseur sur la surface du corps où s'applique le massage. On cherche également souvent à améliorer la douceur de ce massage et à éviter tout effet d'inflammation, en particulier en évitant toute irritation de la peau lors de massages répétés ou longs. Il est courant d'utiliser à cette fin des produits adjuvants tels que des talcs plus ou moins élaborés destinés à faciliter ce massage.
Dans le cadre de ses recherches sur de nouveaux moyens d'amélioration de conditions pathologiques par la seule intervention d'une action mécanique, l'inventeur de la présente invention a maintenant découvert que les lipides peroxydés pouvaient être utilisés de façon particulièrement efficace en tant qu'agents destinés à former un film lipidique à la surface de la peau et que la formation de ce film lipidique permettait, de façon remarquable, d'améliorer un certain nombre de conditions pathologiques, en particulier en permettant, par un effet protecteur, une amélioration de la cicatrisation des plaies, le traitement de différents érythèmes. Plus généralement, il s'est avéré que le film lipidique formé par des lipides peroxydés était particulièrement efficace dans toutes les applications où l'on cherche à isoler la peau du milieu extérieur pour améliorer ses qualités, en particulier ses qualités biomécaniques.
Il s'est également avéré que les lipides peroxydés pouvaient être utilisés comme adjuvant mécanique de massage et cela, en liaison avec la formation d'un film particulièrement efficace formé sur la partie du corps à masser avant ou au cours du massage.
Ce type de modes d'action s'avère particulièrement intéressant à une époque où l'on recherche des modes de traitement aussi peu agressifs que possible pour l'organisme. De tels traitements permettent, en outre, de satisfaire à la réglementation de la Communauté Européenne concernant les dispositifs médicaux, puisque ces derniers couvrent dans un chapitre particulier des produits permettant d'escompter des bénéfices sur la santé associés à des produits d'usage local par le seul effet mécanique lié à leur emploi.
On connaît différents lipides peroxydés, notamment obtenus par peroxydation d'huiles végétales naturelles. On citera, en particulier, les brevets suivants BSM N°2 330 M, EP-A-293 535, FR-A-2 591 112, EP-A-225 831, EP-A-225 832, EP-A-225 833, EP-A-226 506, FR-A-2 461 744, FR-A-2 539 142 et EP-A-117 962 qui concernent soit la préparation de tels lipides peroxydés soit leurs applications dans différents domaines, en particulier dans le traitement de certaines affections dans le domaine de la rhumatologie ou de la traumatologie, ou encore en tant que produit cicatrisant.
Il a maintenant été découvert que ces lipides peroxydés pouvaient être utilisés de façon particulièrement efficace dans toutes les applications où l'on cherche à former un film lipidique à la surface de la peau.
Ainsi, selon l'une de ses caractéristiques essentielles, l'invention concerne l'utilisation pour la fabrication d'une composition à usage topique destinée à former un film à la surface de la peau, de lipides peroxydés en tant qu'agents filmogènes lipidiques.
Les lipides peroxydés dont l'utilisation est revendiquée selon la présente invention résultent de la peroxydation de corps gras insaturés.
Le degré de peroxydation est mesuré selon la nonne ISO 3960.
Pour la mise en oeuvre de la présente invention, on choisira avantageusement des lipides peroxydés présentant un taux de peroxydation compris entre 5 et 600 milli-équivalents par kilo, de préférence entre 30 et 500 milli-équivalents par kilo.
Ce taux de peroxydation sera de façon encore plus avantageuse compris entre 50 et 300 milli-équivalents par kilo.
Les lipides peroxydés préférés utilisés selon l'invention résultent de la peroxydation de lipides ou corps gras d'origine naturelle, de préférence d'origine végétale, de préférence encore de lipides issus d'une huile végétale naturelle.
A titre d'exemples d'huile naturelle choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira l'huile de maïs peroxydée et tout particulièrement une huile de maïs présentant un taux de peroxydation compris entre 5 et 600 milli-équivalents par kilo.

Les lipides peroxydés utilisés selon l'invention sont d'une façon avantageuse constitués, à titre de constituants majoritaires, représentant généralement au moins 80 % de la masse de triglycérides répondant à la formule

CH2-O-R
|
CH-O-R
|
CH2-O-R

dans laquelle les radicaux R sont majoritairement représentés par des acides insaturés en C18 partiellement peroxydés (en fonction du taux de peroxydation dudit lipide).

Les compositions de l'invention peuvent être sous toute forme compatible avec une application topique à la surface de la peau. Ces compositions se trouveront de préférence sous la forme d'une émulsion de type huile-dans-eau ou eau-dans-huile, d'une crème, d'une pommade, d'un lait ou d'un gel, en particulier d'un gel huileux à base de silice colloïdale.

D'une façon générale, les compositions de l'invention comprendront de 2 à 99 % de lipides peroxydés. 'toutefois, leur forme ainsi que leurs compositions dépendront de la zone du corps à traiter et de l'application visée.

Selon un premier aspect de l'invention, le film formé à la surface de la peau a essentiellement un rôle protecteur et isolant permettant, en particulier, d'isoler une plaie ou une lésion cutanée ou une partie inflammée de la peau en vue d'améliorer les conditions de sa cicatrisation ou de sa guérison spontanée.

Parmi les applications pour lesquelles la présente invention s'avère particulièrement intéressante, on citera le traitement des plaies, en particulier des plaies superficielles de la peau. Dans ce type de traitement, la composition contenant les lipides peroxydés agit comme un véritable pansement permettant d'isoler la plaie du milieu extérieur et de favoriser ainsi sa cicatrisation naturelle.

Un autre exemple de conditions pathologiques pour lesquelles les compositions contenant des lipides peroxydés s'avèrent particulièrement intéressantes est celui des brûlures, en particulier des brûlures légères ou des érythèmes légers, en particulier de l'érythème solaire.

L'érythème est une rougeur de la peau sans lésion véritable, souvent provoqué par une cause physique, c'est le cas en particulier des coups de soleil. Mais son origine peut aussi être chimique ou médicamenteuse ou infectieuse ou dermatologique.

Les compositions de l'invention contenant des lipides peroxydés s'avèrent spécialement adaptées à la prévention et au traitement des érythèmes légers localisés, en particulier des érythèmes sans lésion et sans plaie.

Les compositions de l'invention s'avèrent également particulièrement intéressantes dans le cas du traitement de l'érythème solaire et plus précisément de l'érythème solaire localisé.

Dans le cas de ce traitement particulier de l'érythème solaire, il s'est avéré que le type de peau des sujets traités est sans influence sur les résultats, que par ailleurs la tolérance des produits est excellente dans 100 % des cas. Par ailleurs, le produit a été jugé d'utilisation particulièrement facile.

Un autre exemple particulièrement intéressant d'utilisation des compositions de l'invention est celui du traitement de la dermite fessière du nourrisson où l'on a pu observer des taux de guérison particulièrement élevés en fin de traitement et une amélioration très nette des lésions initiales.

Selon un autre aspect, le film réalisé à la surface de la peau grâce à la présence des lipides peroxydés dans la composition a des propriétés essentiellement mécaniques, éventuellement combinées à un effet protecteur.

Ainsi, selon une autre variante particulièrement intéressante de l'invention, il a été mis en évidence que le film lipidique qui se forme à la surface de la peau lors de l'application d'une composition contenant des lipides peroxydés facilite grandement les opérations de massage et améliore la qualité de ce massage.

En effet, l'action de la physiothérapie sur les phénomènes douloureux de l'appareil locomoteur est bien connue. Il est également bien connu que la qualité d'un massage est liée aux gestes du thérapeute aussi bien qu'aux produits adjuvants qu'il emploie pour une meilleure glisse ou une meilleure préhension de son acte de physiothérapie. Tous les praticiens s'accordent à constater qu'un excellent produit de massage peut contribuer à une action antalgique sans commune mesure avec un produit de massage conventionnel.

Ainsi, des essais réalisés par l'inventeur de la présente invention ont permis de démontrer la supériorité des huiles peroxydées par rapport à des huiles minérales et, cela, dans une large gamme de traitements de physiothérapie

orientée vers une action antalgique par massage. Les essais réalisés par l'inventeur ont clairement démontré que les huiles peroxydées constituent un adjuvant de massage particulièrement efficace et bien supérieur aux adjuvants de massages traditionnels. Cette supériorité des adjuvants de massage constitués d'une composition à base de lipides peroxydés par rapport aux produits classiques de massage s'explique, en grande partie, par les propriétés intrinsèques de glisse conférées aux compositions par la présence des huiles peroxydées du fait de la formation du film à la surface de la partie du corps à masser. Cette propriété s'est avérée particulièrement adaptée à l'action mécanique du massage, même dans le cas de massages prolongés ou réitérés, par effleurage, pression ou friction sur des zones corporelles sensibles.

Les compositions à base de lipides peroxydés s'avèrent particulièrement intéressantes quel que soit le type de massage visé. Les compositions à base de lipides peroxydés constituent, en particulier, de remarquables adjuvants de massage qu'il s'agisse aussi bien de massage chauffant. décontractant, ou décongestionnant.

Cette propriété mécanique s'ajoute aux autres actions bénéfiques de l'huile peroxydée, en grande partie elles aussi liées à la qualité du film formé à la surface de la peau, pour contribuer à un résultat du massage particulièrement efficace.

En effet, l'amélioration des caractères de glisse conférés aux compositions à base de lipides peroxydés par la présence de ces produits s'ajoute aux autres avantages conférés eux aussi par la présence des lipides peroxydés dans la composition, en particulier à leur effet sur l'amélioration de l'hydratation de la peau et le renforcement de la résistance cutanée. Cette combinaison de propriétés contribue à améliorer les qualités du massage.

Comme exposé précédemment, le remarquable effet des compositions à base de lipides peroxydés est lié à la qualité essentiellement mécanique du film formé grâce à la présence des lipides à la surface de la peau.

Les compositions de l'invention peuvent être sous toute forme galénique permettant une bonne formation de ce film à la surface de la peau.

La forme pharmaceutique de la composition (gel, crème, solution huileuse, émulsion, etc.) et sa concentration en lipides peroxydés seront choisies en fonction du traitement envisagé et du mode d'application visé.

Par exemple, l'efficacité des compositions dans le massage est particulièrement accentuée avec des formulations de type huile sèche, crème ou lait.

Par ailleurs, les concentrations en lipides peroxydés peuvent être comprises entre 2 et 99 % en poids par rapport au poids de la composition. Toutefois, on préférera des concentrations comprises entre 2 et 5 % pour les lotions, entre 2 et 25 % pour les crèmes, entre 2 et 99 % pour les gels et les solutions huileuses.

Par ailleurs, les taux de peroxydation des lipides peroxydés seront choisis de préférence entre

- 5 et 250 milli-équivalents par kg pour les lotions,
- 5 et 300 milli-équivalents par kg pour les crèmes,
- 5 et 350 milli-équivalents par kg pour les gels et les solutions huileuses.

EXEMPLES

Sauf indication contraire, les proportions données dans les exemples ci-dessous sont exprimées en pourcentage en poids.

Exemple 1 : Crème destinée au traitement des rougeurs, brûlures légères et érythèmes légers localisés

- Triesters de glycérol oxydés à des taux de peroxydation compris entre 5 et 250 milli-équivalents par kg : 20 %
- copolymère acrylique anionique en dispersion dans une huile blanche : 4 %
- parfum : 0,5 %
- parahydroxybenzoate de méthyle sodé : 0,15 %
- parahydroxybenzoate de propyle sodé : 0,05 %
- méthylchloroisothiazolinone et méthylisothiazolinone : 0,0012 %
- eau déminéralisée : qsp 100.

Exemple 2 : Crème destinée au traitement de la dermite fessière irritative du nourrisson

- Triesters de glycérol oxydés à des taux de peroxydation compris entre 5 et 250 milli-équivalents par kg : 25 %
- copolymère acrylique anionique en dispersion dans une huile blanche : 4 %

- parfum : 0,5 %
- parahydroxybenzoate de méthyle sodé : 0,15 %
- parahydroxybenzoate de propyle sodé : 0,05 %
- méthylchloroisothiazolinone et méthylisothiazolinone : 0,0012 %
- eau déminéralisée : qsp 100.

Une étude ouverte non comparative a été menée sur 113 nourrissons âgés de 2 à 14 mois à l'aide du produit ci-dessus utilisé en monothérapie, ad libitum, pendant une à deux semaines. Cette étude a permis de montrer que l'application de la crème ci-dessus permettait d'observer la guérison ou au moins une très nette amélioration dans 82 % des cas traités.

Exemple 3 : lait destiné au traitement de l'érythème solaire

Un lait présentant la composition ci-dessous

- eau déminéralisée : qsp 100
- triesters de glycérol oxydés avec un taux de peroxydation compris entre 5 et 100 milli-équivalents par kg : 20 %
- alcool cétostéarylique éthoxylé 12 : 2 %
- alcool cétostéarylique éthoxylé 20 : 2 %
- glycérine : 1 %
- parfum : 0,3 %
- triéthanolamine : 0,27 %
- polymère acrylique réticulé : 0,2 %
- parahydroxybenzoate de méthyle : 0,15 %
- parahydroxybenzoate de propyle : 0,05 %
- méthylchloroisothiazolinone et méthylisothiazolinone : 0,0012 % a été testé dans le cadre d'une étude ouverte non comparative sur 47 sujets âgés de 2 à 62 ans dans les conditions suivantes
- mode d'application : 1 à 6 applications par jour,
- critères de jugement : amélioration ou disparition du degré de sévérité (notée de 1 à 4) de l'érythème et des signes fonctionnels, acceptabilité du produit.

Les résultats après 48 heures de traitement sont les suivants :

* Degré de sévérité de l'érythème

| Evolution/degré de sévérité | Degré 1 | Degré 2 | Degrés 3 et 4 |
|---|---|---|---|
| Disparition | 68 % | 38 % | --- |
| Atténuation | 32 % | 72 % | 100 % |

* Signes fonctionnels

| Evolution/degré de sévérité | Sensation de chaleur | Sensation de picotement | Sensation de cuisson (burning) |
|---|---|---|---|
| Disparition | 74 % | 75 % | 61 % |
| Atténuation | 26 % | 22 % | 22 % |

* Incidence du type de peau des sujets

Le type de peau déterminé à l'inclusion selon la classification de FITZ PATRICK n'a pas d'influence sur les résultats.

* Tolérance et acceptabilité du produit

La tolérance a été excellente dans 100 % des cas et le produit a été jugé d'utilisation facile dans 96 % des cas et d'usage agréable dans 93 % des cas.

* Conclusion

Ces résultats illustrent l'intérêt de la crème à base de triglycérides oxydés dans le traitement de l'érythème solaire localisé.

Exemple 4 : Mise en évidence de l'effet des huiles peroxydées en tant qu'adjuvant de massage

Une étude a été réalisée sur 50 patients en vue de comparer une composition à base d'huile végétale peroxydée avec une huile minérale dans une large gamme de traitements de physiothérapie orientée vers une action antalgique par massage.

Les deux produits testés sont sous la forme de baumes liquides comprenant respectivement :

- produit selon l'invention : 93 % de glycérides oxydés et 7 % de matière parfumante,
- produit placebo : 93 % d'huile minérale et 7 % de matière parfumante.

a) Protocole du test

L'efficacité et la tolérance clinique des produits de l'invention ont été testées au cours d'un essai réalisé en double aveugle en comparaison avec une huile minérale.

Les critères d'inclusion dans cette étude étaient les suivants : sujets adultes des deux sexes présentant diverses manifestations algiques, avec ou sans état inflammatoire, de l'appareil locomoteur.

Aucun critère d'exclusion n'a été retenu dans cet essai en dehors de l'allergie cutanée.

Les deux produits testés étaient contenus dans des flacons de présentation strictement identique, numérotés selon une liste de randomisation et attribués aux sujets dans l'ordre chronologique de leur entrée dans l'essai.

Le praticien a limité son geste thérapeutique à deux massages par jour. Le traitement devait être poursuivi pendant 15 jours, selon l'évolution des signes cliniques. Aucun antalgique ou anti-inflammatoire ne devait être associé au massage par l'huile peroxydée ou au massage par l'huile minérale pendant toute la durée de l'essai.

Un interrogatoire et un examen clinique des sujets devaient permettre de juger de l'efficacité du traitement, par notation de l'évolution de l'ensemble de la symptomatologie clinique (0 : résultat nul, + : résultat moyen, ++ : résultat bon, +++ : résultat excellent) et de préciser la tolérance clinique des produits.

La durée moyenne du traitement a varié de 48 heures à 8 jours en fonction du résultat obtenu.

b) Résultats

Les résultats sont rassemblés dans le tableau ci-dessous.

| | Nombre de malades | |
|---|---|---|
| | Sous massage avec huile peroxydée | Sous massage avec huile minérale |
| Résultats excellents | 11 (44 %) | 4 (16 %) |
| Résultats bons | 13 (52 %) | 8 (32 %) |
| Résultats légers, nuls et aggravation | 1 (4 %) | 13 (52 %) |

Soit, résultats très satisfaisants : (excellents et bons).

- 96 % dans le groupe sous massage avec l'huile peroxydée
- 48 % dans le groupe sous massage avec l'huile minérale.

Calculs statistiques :

X2 (corrigé de yates) = 15,22 ; ddl = 2 ; $\alpha$ > 0,001

Ainsi, les résultats obtenus sont hautement significatifs en faveur de l'huile hyperoxygénée.

Délai d'action :

Il est inférieur ou égal à 10 jours dans :

- 72 % des cas traités par massage avec l'huile hyperoxygénée
- 40 % des cas traités par massage avec l'huile minérale.

**Revendications**

**1.** Utilisation pour la fabrication d'une composition à usage topique destinée à former un film lipidique sur la peau, de lipides peroxydés en tant qu'agents filmogènes lipidiques.

**2.** Utilisation selon la revendication 1, caractérisée en ce que ledit film est destiné à améliorer la cicatrisation d'une plaie.

**3.** Utilisation selon l'une des revendications 1 ou 2, caractérisée en ce que ledit film est destiné au traitement des brûlures légères ou des érythèmes légers, en particulier de l'érythème solaire.

**4.** Utilisation selon l'une des revendications 1 ou 2, caractérisée en ce que ladite composition est destinée au traitement de la dermite fessière du nourrisson, en particulier de l'érythème fessier du nourrisson.

**5.** Utilisation selon la revendication 1, caractérisée en ce que ledit film lipidique est destiné à améliorer les qualités mécaniques d'un massage.

**6.** Utilisation selon l'une des revendications 1 à 5, caractérisée en ce que lesdits lipides peroxydés présentent un taux de peroxydation compris entre 5 et 600 milli-équivalents par kg, de préférence entre 30 et 500 milli-équivalents par kg, de préférence encore entre 50 et 300 milli-équivalents par kg.

**7.** Utilisation selon l'une des revendications 1 à 6, caractérisée en ce que lesdits lipides peroxydés comprennent, à titre de constituants majoritaires, des triglycérides partiellement oxydés répondant à la formule générale :

$$\begin{array}{l} CH_2\text{-}O\text{-}R \\ | \\ CH\text{-}O\text{-}R \\ | \\ CH_2\text{-}O\text{-}R \end{array}$$

dans laquelle les radicaux R sont des acides insaturés en C18 partiellement peroxydés.

**8.** Utilisation selon l'une des revendications 1 à 7, caractérisée en ce que lesdits lipides peroxydés sont obtenus par peroxydation de lipides d'origine végétale, de préférence de lipides issus d'une huile végétale naturelle.

**9.** Utilisation selon la revendication 8, caractérisée en ce que l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

**10.** Utilisation selon l'une des revendications 1 à 9, caractérisée en ce que ladite composition contient de 2 à 99 % en poids d'huile peroxydée.

**11.** Utilisation selon l'une des revendications 1 à 10, caractérisée en ce que ladite composition est sous la forme d'une émulsion de type huile-dans-eau ou eau-dans-huile, d'une crème, d'une pommade, d'un lait ou d'un gel, en particulier d'un gel huileux à base de silice colloïdale.

Office européen des brevets

# RAPPORT DE RECHERCHE EUROPEENNE

Numéro de la demande
EP 00 40 2277

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | FR 2 591 104 A (DESJONQUERES)<br>12 juin 1987 (1987-06-12)<br>* le document en entier *<br>--- | 1-11 | A61K7/48<br>A61K35/78 |
| A,D | EP 0 117 962 A (LEPELTIER ET AL.)<br>12 septembre 1984 (1984-09-12)<br>* le document en entier *<br>--- | 1-11 | |
| A | FR 2 591 113 A (DESJONQUERES)<br>12 juin 1987 (1987-06-12)<br>* le document en entier *<br>----- | 1-11 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| LA HAYE | 11 décembre 2000 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 00 40 2277

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-12-2000

| Document brevet cité au rapport de recherche | | Date de publication | Membre(s) de la famille de brevet(s) | | Date de publication |
|---|---|---|---|---|---|
| FR 2591104 | A | 12-06-1987 | AUCUN | | |
| EP 117962 | A | 12-09-1984 | FR | 2539142 A | 13-07-1984 |
| | | | FR | 2555051 A | 24-05-1985 |
| | | | AT | 29735 T | 15-10-1987 |
| | | | DE | 3373675 D | 22-10-1987 |
| FR 2591113 | A | 12-06-1987 | AUCUN | | |

EPO FORM P0460

Pour tout renseignement concernant cette annexe : voir Journal Officiel de l'Office européen des brevets, No.12/82